# EUROPEAN PATENT SPECIFICATION

(11) **EP 3 930 482 B1**
(45) Date of publication and mention of the grant of the patent: **02.07.2025**
(21) Application number: 20713738.1
(22) Date of filing: 25.02.2020
(51) Int. Cl.: A61K 33/06, A61K 33/14, A61K 35/20, A61P 3/02, A23L 29/30, A23L 33/125, A23L 33/21, A23L 33/00, A61K 31/702, A61K 45/06, A61P 1/00

(54) **COMPOSITION COMPRISING HUMAN MILK OLIGOSACCHARIDE FOR USE IN SUPPORTING AND/OR IMPROVING INTESTINAL HEALTH**
ZUSAMMENSETZUNG ENTHALTEND MENSCHLICHES MILCH-OLIGOSACCHARID ZUR VERWENDUNG ZUR UNTERSTÜTZUNG UND/ODER VERBESSERUNG DER DARMGESUNDHEIT
COMPOSITION COMPRENANT UN OLIGOSACCHARIDE DU LAIT HUMAIN POUR UTILISATION DANS LE SOUTIEN ET/OU L'AMÉLIORATION DE LA SANTÉ INTESTINALE

(30) Priority: 25.02.2019 BE 201905116; 05.03.2019 BE 201905134
(43) Date of publication of application: 05.01.2022
(73) Proprietor: Nutribam BV, 2390 Malle (BE)
(72) Inventor: VAN RIET, Nikolaas, 3545 Halen (BE)
(74) Representative: Brantsandpatents bv
(86) International application number: PCT/IB2020/051589
(87) International publication number: WO 2020/174386

(56) References cited:
- EP-A1- 2 465 507
- EP-A1- 2 896 628
- WO-A1-2011/136637
- WO-A1-2013/154725
- WO-A1-2014/100696
- WO-A1-2017/046711
- WO-A1-2017/103019
- WO-A1-2017/144062
- WO-A1-2017/190754
- WO-A1-2018/020473
- WO-A1-2018/024870
- WO-A1-2018/187792
- ZACHERY T LEWIS ET AL: "Maternal fucosyltransferase 2 status affects the gut bifidobacterial communities of breastfed infants", MICROBIOME, BIOMED CENTRAL LTD, LONDON, UK, vol. 3, no. 1, 10 April 2015 (2015-04-10), pages 13, XP021215967, ISSN: 2049-2618, DOI: 10.1186/S40168-015-0071-Z

## Description

### TECHNICAL FIELD

The invention relates to sport performance. In particular, the invention is in the technical area of compositions for supporting and/or improving sports performance in endurance athletes.

### PRIOR ART

Regular exercise and/or sport offers many benefits, including protection against the development of chronic diseases and improved quality of life. These benefits are attributed, among other things, to the anti-inflammatory effects and the strengthening of the neuromuscular function associated with exercise. In addition, intensive sports practice leads to changes in the gastrointestinal microbiota, which is closely related to the sportsperson's well-being. For example, the gastrointestinal microbiota play an important role in the production, storage and consumption of energy obtained from the diet, as well as in inflammation, redox reactions and hydration status.

However, the correlation between exercise and the gastrointestinal microbiota can also be detrimental to persons engaged in sports, especially elite athletes. For example, long and intensive training sessions may lead to the disruption of the gastrointestinal microbiota. As a result, the sportsperson delivers significantly less sports performance and his recovery capacity is drastically reduced. A disrupted gastrointestinal microbiota also reduces the functioning of the immune system, which puts athletes at greater risk of illness, infections, in particular intestinal disorders. In addition, athletes who regularly consume food supplements, especially supplements rich in fructose and/or artificial sweeteners, are at an increased risk of dysregulated gastrointestinal microbiota and infection as these components reach the intestinal tract intact and function as potential nutrients for pathogenic bacteria.

Accordingly, there is a need to support and/or improve intestinal health in athletes in order to support the immune system, minimise the risk of infection, and optimise athletes' sports performance and recovery capacity.

WO 2017 180 501 describes a probiotic composition for improving sports performance and recovery. However, the prebiotic composition of WO '501 has only limited influence on the athlete's immune system and the risk of infections related to the occurrence of pathogenic bacteria in the gastrointestinal tract is little or not affected.

WO 2018 187 792 discloses a 2'-fucosyllactose component and a method of using said component for the treatment of inflammatory intestinal disorders, such as Crohn's disease and ulcerative colitis, or to reduce the risk of relapse in inflammatory intestinal disorders. WO '792, however, emphasises inflammatory intestinal disorders. No indication is given as to how 2'-fucosyllactose can be used non-therapeutically to improve and/or support intestinal health in athletes undertaking long-term and intensive sports performance.

Furthermore, WO 2014/100696 and WO 2018/020473 describe human milk oligosaccharides (HMOs) in relation to intestinal health and cognitive functions. WO 2018/187792 focuses on the use of HMOs in Inflammatory Bowel Disease (IBD). WO 2017/064711 and WO 2017/144062 describe the relationship between HMOs and the intestinal flora, specifically with regard to certain microorganisms, as well as the treatment of intestinal disorders and other disorders. WO 2013/154725 and WO 2017/190754 also describe the use of HMOs in persons with intestinal disorders. WO 2018/024870 and WO 2017/103019 focus on the benefits of HMOs in young children. EP 2 896 628 and EP 2 465 507 discuss the purification of HMOs and describe some applications. None of these documents, however, establish a clear link between HMOs, the athlete and their sports performance.

The object of the present invention is to at least solve some of the above-mentioned problems or disadvantages.

### SUMMARY OF THE INVENTION

To this end, the invention provides a method for supporting and/or improving sports performance and/or non-therapeutic recovery in athletes by administering a composition according to claim 1. The composition comprises an effective dose of at least one human milk oligosaccharide (HMO) selected from the group of 2'-fucosyllactose (2'-FL), 3-fucosyllactose (3-FL), lactodifucotetraose (DF-L), lacto-N-tetraose (LNT), lacto-N-neotetraose (LNnT), lacto-N-fucopentaose I (LNFP I), lacto-N-difucohexaose II (LNDFH II), lacto-N-neodifucohexaose II (LNnDFH II), para lacto-N-neohexaose (para-LNnH), lacto-N-neooctaose (LNnO), lacto-N-fucopentaose V (LNFP V), lacto-N-neofucopentaose V (LNnFP V), 3'-sialyl-3-fusoyl lactose (F-SL), LS-tetrasaccharide a (LSTa), 3'-sialyllactose (3'-SL), or combinations thereof.

Preferred embodiments of the method are given in dependent claims 2 to 10.

### DETAILED DESCRIPTION

Unless otherwise defined, all terms used in the description of the invention, including technical and scientific terms, have the meaning as commonly understood by a person skilled in the art to which the invention pertains. For a better understanding of the description of the invention, the following terms are explained explicitly.

In this document, 'a' and 'the' refer to both the singular and the plural, unless the context presupposes otherwise. For example, 'a segment' means one or more segments.

When the term 'around' or 'about' is used in this document with a measurable quantity, a parameter, a duration or moment, and the like, then variations are meant of approx. 20% or less, preferably approx. 10% or less, more preferably approx. 5% or less, even more preferably approx. 1% or less, and even more preferably approx. 0.1% or less than and of the quoted value, insofar as such variations are applicable in the described invention. However, it must be understood that the value of a quantity used where the term 'about' or 'around' is used, is itself specifically disclosed.

The terms 'comprise', 'comprising', 'consist of', 'consisting of', 'provided with', 'include', 'including', 'contain', 'containing', are synonyms and are inclusive or open terms that indicate the presence of what follows, and which do not exclude or prevent the presence of other components, characteristics, elements, members, steps, as known from or disclosed in the prior art.

Quoting numerical intervals by endpoints comprises all integers, fractions and/or real numbers between the endpoints, these endpoints included.

The present invention relates to a method for supporting and/or improving sports performance and/or non-therapeutic recovery in endurance athletes, wherein the athlete is a secretor or non-secretor of an human milk oligosaccharide (HMO), preferably 2'-fucosyllactose (2'-FL), and wherein the athlete is administered a composition comprising an effective dose of at least one HMO selected from the group of 2'-fucosyllactose (2'-FL), 3-fucosyllactose (3-FL), lactodifucotetraose (DF-L), lacto-N-tetraose (LNT), lacto-N-neotetraose (LNnT), lacto-N-fucopentaose I (LNFP I), lacto-N-difucohexaose II (LNDFH II), lacto-N-neodifucohexaose II (LNnDFH II), para lacto-N-neohexaose (para-LNnH), lacto-N-neooctaose (LNnO), lacto-N-fucopentaose V (LNFP V), lacto-N-neofucopentaose V (LNnFP V), 3'-sialyl-3-fusoyl lactose (F-SL), LS-tetrasaccharide a (LSTa), 3'- sialyllactose (3'-SL), or combinations thereof, characterised in that the effective dose of the HMO in a non-secretor is comprised between 1.8 and 4.0 g/day and characterised in that the effective dose of the HMO in a secretor is comprised between 0.8 and 3.0 g/day.

In the context of the present invention, the term 'human milk oligosaccharides' (HMOs) refers to sugar molecules that occur naturally in human breast milk. HMOs have a prebiotic effect in the newborn child and are essential for the development of a healthy intestinal flora. HMOs in the context of the present invention are available from both a natural and synthetic source.

The term 'athlete' can be interpreted as any individual who engages in sports activities. Sports activities according to the present invention comprise activities from the group of, but are not limited to, swimming, football, karate, hockey, strength training, weightlifting, shot-putting, gymnastics, athletics, cycling, running, sprinting, hurdling, skating, rowing, long jump, boxing, skiing, anaerobic training, aerobics, or combinations thereof.

According to an embodiment, the composition further comprises one or more excipients, wherein the HMO and the excipients are in a ratio comprised between 1:8 and 6:8. In this light, the composition can also be interpreted as a food supplement, or sports supplement. A ratio of 1:8 to 6:8 allows the HMO, and in particular the 2'-FL, to act optimally towards one or more of its effects, the one or more excipients providing the composition with additional, beneficial effects. Moreover, within the present ratios, the composition forms little to no additional burden on the gastrointestinal system and is optimally absorbed by the subject. Preferably, the 2'-fucosyllactose and the one or more excipients are in a ratio comprised between 1:8 and 3:8. The present proportions allow the addition of more excipients to the composition, whereby several additional effects are obtained.

According to an embodiment, the one or more excipients are selected from the group of amino acids, monosaccharides, micronutrients, antioxidants, vitamins, vegetable extracts, fibres, derivatives of any of the previous excipients, or combinations thereof.

According to an embodiment, the vitamins are selected from the group of vitamin A, vitamin D, vitamin E, vitamin K, thiamine, riboflavin, pyridoxine, vitamin C, carotenoids, niacin, folic acid, pantothenic acid, biotin, choline, inositol, their salts and derivatives, and combinations thereof. The excipients according to the present invention contribute to the proper functioning of the gastrointestinal system, support intestinal health and support the general well-being of the subject, in particular the athlete.

According to an embodiment, the present invention comprises vegetable extracts selected from the group of ginger extract, soy extract, garlic extract, *echinacea* extract, ginseng extract, St. John's wort extract, elderberry extract, cranberry extract, turmeric extract, *Ginkgo biloba* extract, or combinations thereof. They are a natural source of polyphenols, and may act as an antioxidant and/or support the immune system and athletic performance. Preferably, the composition comprises cranberry extract. The use of cranberry extract in the present composition is particularly advantageous since cranberry extract acts as proanthocyanidin and is capable of triggering beneficial bacterial strains such as *Akkermansia muciniphila.*

According to some embodiments, the composition comprises one or more polyphenols, preferably proanthocyanidins, to stimulate the growth of *Akkermansia muciniphila* in the intestinal microbiome. This addition influences, in particular by the said proliferation of *Akkermansia,* various metabolic processes, with a favourable effect on the body composition. In particular, the proliferation of *Akkermansia* was associated with maintaining a low fat percentage. Considering the sports target group of the present invention, this is an important additional advantage of the composition. In the context of the present invention, the amino acids preferably comprise glutamine, and branched-chain amino acids (BCAAs), namely leucine, isoleucine and/or valine. During intensive training they can be used directly as an energy source. In addition, they contribute, especially leucine, to activation of muscle protein synthesis. In combination with the HMOs of the present invention, the recovery time is further shortened.

'Antioxidants' are components capable of capturing free radicals and therefore lowering oxidative stress. According to an embodiment, the composition comprises one or more antioxidants suitable for oral administration, including vitamin A, vitamin E, vitamin C, retinol, tocopherol, carotenoids, including lutein, beta-carotene, zeaxanthin, lycopene, and combinations thereof.

According to an embodiment, said micronutrients comprise calcium, phosphorus, sodium, chloride, magnesium, manganese, iron, copper, zinc, selenium, iodine, chromium, molybdenum, or combinations thereof.

The composition further comprises according to an embodiment one or more proteins, carbohydrates and/or fats. According to an embodiment, the composition comprises one or more protein sources, which have a total concentration in the supplement comprised between 40.0 and 80.0 m%, preferably between 50.0 and 80.0 m%, more preferably between 60.0 and 80.0 m%, even more preferably between 65.0 and 80.0 m%.

The composition can be formulated in a form adapted to the chosen route of administration, including, but not limited to, oral or parental, including subcutaneous, intramuscular, intraperitoneal, intratumoral and intravenous administration. 'Oral administration', as defined herein, comprises any form of administration in which the composition is absorbed via the subject's oesophagus. Oral formulations comprise any solid, liquid or powder formulation suitable for use herein, with the proviso that such formulation allows for the safe and effective oral delivery of the effective dose of 2'-fucosyllactose (2'-FL).

According to an embodiment, the composition is a soluble powder. The soluble powder has the advantage that the composition can be stored in a suitable container of only limited size and can be dissolved in a liquid by the consumer. The composition not only takes up a small volume, but it also has a long shelf life.

According to an embodiment, the composition is directly ingestible as a capsule. In another embodiment, the composition is ingestible through food products and/or drinks.

According to a preferred embodiment, the composition is a sports drink. A sports drink brings a great ease of use for the consumer, since no actions are required immediately before ingestion by the consumer.

Preferably, the HMO comprises 2'-fucosyllactose (2'-FL).

According to an embodiment, it is determined whether the athlete is a secretor or non-secretor of an HMO by monitoring for the presence or absence of one or more genetic polymorphisms or mutations in the 2-alpha-L-fucosyl transferase 2 (FUT2) gene.

In another embodiment, the athlete's secretor / non-secretor status is determined by checking for the presence or absence of one or more genetic polymorphisms or mutations in a gene other than the 2-alpha-L-fucosyl transferase 2 (FUT2) gene.

According to a further or alternative embodiment, the secretor / non-secretor status of the athlete is determined by checking for the presence or absence of one or more polymorphisms at the protein level, in particular the protein encoded by the FUT2 gene. The protein encoded by FUT2 is a Golgi membrane stacking protein involved in the production of a precursor of the H antigen required for the final step in the soluble A and B antigen synthesis pathway. This gene is one of two genes encoding the 2-alpha-L-fucosyltransferase 2 enzyme. SNPs at the level of the FUT2 gene and/or polymorphisms in the protein encoded by this gene lead to a reduced to absent secretion of 2'-FL and are therefore extremely suitable for determining the secretor / non-secretor status with regard to 2'-FL.

More preferably, the method comprises a DNA analysis, wherein single nucleotide polymorphisms (SNPs) in the 2-alpha-L-fucosyltransferase 2 (FUT2) gene are identified. The FUT2 gene is involved in the production of 2'-FL, where individuals with an inactivating polymorphism in the FUT2 gene are called 'non-secretors'. Non-secretors are deficient in innate intestinal carbohydrates that contain fucose, which makes them extra susceptible to intestinal dysbiosis. The term 'intestinal dysbiosis' denotes an imbalance between different microorganisms present in the intestinal system. In addition, non-secretors have a higher chance of developing intestinal disorders such as Crohn's disease.

The single nucleotide polymorphisms (SNPs) preferably comprise the SNPs rs601338, rs492602, rs516246 and/or rs602662. Such SNP numbering can be consulted on databases such as SNPedia. The SNPs of the present embodiment are exceptionally relevant for the non-secretor status of a subject with respect to 2'-FL. Preferably SNP rs601338 is detected, of which 25 to 30% of the population is a carrier. More preferably, at least 2 SNPs from the group of rs601338, rs492602 and/or rs602662 are detected. Even more preferably, rs601338, rs492602 and rs602662 are detected. DNA analysis and/or the detection of SNPs according to the present invention is performed by standard techniques as known from the prior art. Preferably, a microarray or a single nucleotide polymorphism (SNP) chip is used to identify SNPs present in the FUT2 gene.

According to an embodiment, the effective dose of the HMO, especially 2'-fucosyllactose (2'-FL), in a non-secretor is preferably comprised between 2.0 and 3.5 g/day, more preferably between 2.0 and 3.0 g/day.

An embodiment comprises an effective dose of the HMO, especially 2'-fucosyllactose (2'-FL), in a secretor preferably between 1.0 and 2.5 g/day, more preferably comprised between 1.8 and 2.2 g/day.

According to an embodiment of the method, supporting and/or improving sports performance and/or non-therapeutic recovery comprises an increased production of short chain fatty acids (SCFAs) in the intestines. The increased production of short chain fatty acids has the advantage that it produces an increased gene expression in the intestinal wall. The induction of histone hyperacetylation by short chain fatty acids may play a beneficial role in this. Since gene expression in the intestinal wall is closely linked to mitochondrial biogenesis, an increased production of mitochondria in the athlete is also realised by using the present composition. Increased production of mitochondria, which play an essential role in energy production and regulation, is of great added value in athletes, especially endurance athletes. Such a composition is extremely beneficial for supporting and improving sports performance, as well as for improving recovery in an athlete.

According to an embodiment of the method, supporting and/ or improving sports performance and/or non-therapeutic recovery comprises improving synaptic plasticity. This is particularly advantageous for athletes who practice sports of a certain technical level of difficulty and supports them in learning and/or improving complex sports skills.

According to an embodiment of the method, the athlete has followed a course of antibiotics prior to the method. Following a course of antibiotics has an adverse effect on intestinal health, which makes the method according to the present invention particularly advantageous.

### EXAMPLES

The invention will now be further elucidated by means of the following examples, without, however, being limited thereto.

### EXAMPLE 2: composition for optimising recovery in endurance athletes

The composition shown in the table below optimises recovery in athletes by supporting intestinal health from a daily dose of 2.5 g/day.

| ingredient | concentration (m%) |
|---|---|
| 2'-fucosyllactose (2'-FL) | 80.00 |
| various fillers | 20.00 |

In 20 endurance athletes who were administered the composition at 2.5 g/day for 60 days, the required recovery time after intensive training was shortened by 5 to 10% compared to the control group (20 subjects). Athletes who received the composition experienced less abdominal and intestinal complaints during and/or after intensive training and indicated that they felt recovered more quickly.

Twenty-five percent of the athletes tested indicated that they were also better able to schedule intensive training sessions that followed each other in quick succession.

### EXAMPLE 3: Powder-formed food supplement for athletes

The food supplement with composition as shown in the following table is a suitable addition to a healthy diet, improving intestinal health, improving recovery and sports performance.

| ingredient | concentration (m%) |
|---|---|
| 2'-fucosyllactose (2'-FL) | 25.00 |
| micronutrients (Zn salt) | 0.10 |
| L-glutamine | 6.30 |
| vegetable extracts | 65.00 |
| various fillers | 3.60 |

The food supplement is powdery and should be dissolved in water, milk, orange juice or another drinkable liquid. For daily consumption, preferably a dose of 8g is dissolved in a liquid volume of 100 mL.

Daily consumption improves the immune system in athletes, as a result of improved intestinal health. In particular, when other supplements are used, especially supplements comprising fructose and/or artificial sweeteners, optimal gut motility is obtained, and the risk of pathogenic infections is minimised.

### EXAMPLE 4: Protein powder with human milk oligosaccharides

The protein powder with the composition below supports an optimal build-up of muscle mass and optimises the required recovery time between workouts, allowing a higher training volume.

| ingredient | concentration (m%) |
|---|---|
| whey protein | 68.00 |
| carbohydrates | 16.00 |
| 2'-fucosyllactose (2'-FL) | 4.50 |
| calcium (Ca) | 0.30 |
| potassium (K) | 0.40 |
| various additives | 10.80 |

With daily consumption of 45g, preferably immediately after a strength training, the build-up of muscle mass is maximised and the negative side-effects of an intensive training session on the gastrointestinal tract minimised. The protein powder is suitable for a solution of 200 to 300 mL of drinkable liquid. The present powder shortens the required recovery time after a workout, which is particularly advantageous if a training regimen of 4 to 6 sessions per week is envisaged.

## Claims

1. A method for supporting and/or improving sports performance and/or non-therapeutic recovery in endurance athletes, wherein the athlete is a secretor or non-secretor of an human milk oligosaccharide (HMO), preferably 2'-fucosyllactose (2'-FL), and wherein the athlete is administered a composition comprising an effective dose of at least one HMO selected from the group of 2'-fucosyllactose (2'-FL), 3-fucosyllactose (3-FL), lactodifucotetraose (DF-L), lacto-N-tetraose (LNT), lacto-N-neotetraose (LNnT), lacto-N-fucopentaose I (LNFP I), lacto-N-difucohexaose II (LNDFH II), lacto-N-neodifucohexaose II (LNnDFH II), para lacto-N-neohexaose (para-LNnH), lacto-N-neooctaose (LNnO), lacto-N-fucopentaose V (LNFP V), lacto-N-neofucopentaose V (LNnFP V), 3'-sialyl-3-fusoyl lactose (F-SL), LS-tetrasaccharide a (LSTa), 3'-sialyllactose (3'-SL), or combinations thereof, **characterised in that** the effective dose of the HMO in a non-secretor is comprised between 1.8 and 4.0 g/day and **characterised in that** the effective dose of the HMO in a secretor is comprised between 0.8 and 3.0 g/day.

2. The method according to claim 1, wherein the human milk oligosaccharide (HMO) is a synthetically produced HMO.

3. The method according to any of the preceding claims 1 or 2, wherein supporting and/or improving sports performance and/or non-therapeutic recovery comprises an increased production of short chain fatty acids (SCFA) in the intestines.

4. The method according to any of the preceding claims 1 to 3, wherein the athletes have followed a course of antibiotics prior to administration of the composition.

5. The method according to any of the preceding claims 1 to 4, wherein the athletes are tested for the presence or absence of one or more genetic polymorphisms or mutations prior to administration of the composition.

6. The method according to claim 5, wherein said polymorphisms or mutations concern the FUT2 gene.

7. The method according to any one of the preceding claims 1 to 6, wherein the composition comprises one or more excipients, wherein the HMO and the one or more excipients relate according to a ratio which is comprised between 1:8 and 6:8, preferably between 1:8 and 3:8.

8. The method according to claim 7, wherein the one or more excipients are selected from the group of amino acids, monosaccharides, micronutrients, vegetable extracts, fibres, derivatives of any of the preceding excipients, or combinations thereof.

9. The method according to any of the preceding claims 1 to 8, wherein the composition is a soluble powder.

10. The method according to any of the preceding claims 1 to 9, wherein the composition is a sports drink.

## Patentansprüche

1. Ein Verfahren zum Unterstützen und/oder Verbessern der sportlichen Leistung und/oder der nicht-therapeutischen Wiederherstellung von Ausdauersportlern, wobei der Sportler ein Ausscheider oder Nicht-Ausscheider eines humanen Milch-Oligosaccharids (HMO), vorzugsweise 2'-Fucosyllactose (2'-FL), ist und wobei dem Sportler eine Zusammensetzung verabreicht wird, die eine wirksame Dosis mindestens eines HMO umfasst, ausgewählt aus der Gruppe aus 2'-Fucosyllactose (2'-FL), 3-Fucosyllactose (3-FL), Lactodifucotetraose (DF-L), Lacto-N-tetraose (LNT), Lacto-N-neotetraose (LNnT), Lacto-N-fucopentaose I (LNFP I), Lacto-N-difucohexaose II (LNDFH II), Lacto-N-neodifucohexaose II (LNnDFH II), para-Lacto-N-neohexaose (para-LNnH), Lacto-N-neooctaose (LNnO), Lacto-N-fucopentaose V (LNFP V), Lacto-N-neofucopentaose V (LNnFP V), 3'-Sialyl-3-fusoyl-lactose (F-SL), LS-tetrasaccharid a (LSTa), 3'-Sialyllactose (3'-SL) oder Kombinationen daraus, **dadurch gekennzeichnet, dass** die wirksame Dosis des HMO für einen Nicht-Ausscheider zwischen 1,8 und 4,0 g/Tag beträgt, und **dadurch gekennzeichnet, dass** die wirksame Dosis des HMO für einen Ausscheider zwischen 0,8 und 3,0 g/Tag beträgt.

2. Das Verfahren nach Anspruch 1, wobei das humane Milch-Oligosaccharid (HMO) ein synthetisch produziertes HMO ist.

3. Das Verfahren nach einem der vorhergehenden Ansprüche 1 oder 2, wobei das Unterstützen und/oder Verbessern der sportlichen Leistung und/oder der nicht-therapeutischen Wiederherstellung eine erhöhte Produktion von kurzkettigen Fettsäuren (SCFA) im Darm umfasst.

4. Das Verfahren nach einem der vorhergehenden Ansprüche 1 bis 3, wobei sich die Sportler vor dem Verabreichen der Zusammensetzung einer Antibiotika-Behandlung unterzogen haben.

5. Das Verfahren nach einem der vorhergehenden Ansprüche 1 bis 4, wobei die Sportler vor dem Verabreichen der Zusammensetzung auf das Vorhandensein oder das Nicht-Vorhandensein eines/einer oder mehrerer genetischer Polymorphismen oder Mutationen getestet werden.

6. Das Verfahren nach Anspruch 5, wobei die Polymorphismen oder Mutationen das FUT2-Gen betreffen.

7. Das Verfahren nach einem der vorhergehenden Ansprüche 1 bis 6, wobei die Zusammensetzung einen oder mehrere Hilfsstoffe umfasst, wobei das HMO und der eine oder die mehreren Hilfsstoffe in einem Verhältnis zueinander vorhanden sind, das zwischen 1:8 und 6:8, vorzugsweise zwischen 1:8 und 3:8, beträgt.

8. Das Verfahren nach Anspruch 7, wobei der eine oder die mehreren Hilfsstoffe aus der Gruppe aus Aminosäuren, Monosacchariden, Mikronährstoffen, Pflanzenextrakten, Fasern, Derivaten von einem der vorhergehenden Hilfsstoffe oder Kombinationen daraus ausgewählt sind.

9. Das Verfahren nach einem der vorhergehenden Ansprüche 1 bis 8, wobei die Zusammensetzung ein lösliches Pulver ist.

10. Das Verfahren nach einem der vorhergehenden Ansprüche 1 bis 9, wobei die Zusammensetzung ein Sportgetränk ist.

## Revendications

1. Procédé permettant de soutenir et/ou d'améliorer la performance sportive et/ou la récupération non thérapeutique chez des athlètes d'endurance, dans lequel l'athlète est un sécréteur ou un non-sécréteur d'un oligosaccharide du lait humain (HMO), de préférence le 2'-fucosyllactose (2'-FL), et dans lequel l'athlète se voit administrer une composition comprenant une dose efficace d'au moins un HMO choisi dans le groupe constitué par 2'-fucosyllactose (2'-FL), 3-fucosyllactose (3-FL), lactodifucotétraose (DF-L), lacto-N-tétraose (LNT), lacto-N-néotétraose (LNnT), lacto-N-fucopentaose I (LNFP I), lacto-N-difucohexaose II (LNDFH II), lacto-N-néodifucohexaose II (LNnDFH II), para lacto-N-néohexaose (para-LNnH), lacto-N-néooctaose (LNnO), lacto-N-fucopentaose V (LNFP V), lacto-N-néofucopentaose V (LNnFP V), 3'-sialyl-3-fusoyl lactose (F-SL), LS-tétrasaccharide a (LSTa), 3'-sialyllactose (3'-SL), ou leurs combinaisons, **caractérisé en ce que** la dose efficace de HMO chez un non-sécréteur est comprise entre 1,8 et 4,0 g/jour et **caractérisé en ce que** la dose efficace de HMO chez un sécréteur est comprise entre 0,8 et 3,0 g/jour.

2. Procédé selon la revendication 1, dans lequel l'oligosaccharide du lait humain (HMO) est un HMO produit synthétiquement.

3. Procédé selon l'une quelconque des revendications précédentes 1 ou 2, dans lequel le soutien et/ou l'amélioration de la performance sportive et/ou de la récupération non thérapeutique comprennent une production accrue d'acides gras à chaîne courte (AGCC) dans les intestins.

4. Procédé selon l'une quelconque des revendications précédentes 1 à 3, dans lequel les athlètes ont suivi un traitement antibiotique avant l'administration de la composition.

5. Procédé selon l'une quelconque des revendications précédentes 1 à 4, dans lequel les athlètes sont testés relativement à la présence ou à l'absence d'un ou plusieurs polymorphismes ou mutations génétiques avant l'administration de la composition.

6. Procédé selon la revendication 5, dans lequel lesdits polymorphismes ou mutations concernent le gène FUT2.

7. Procédé selon l'une quelconque des revendications précédentes 1 à 6, dans lequel la composition comprend un ou plusieurs excipients, dans lequel le HMO et le ou les excipients sont liés selon un rapport qui est compris entre 1:8 et 6:8, de préférence entre 1:8 et 3:8.

8. Procédé selon la revendication 7, dans lequel le ou les excipients sont choisis dans le groupe constitué par acides aminés, monosaccharides, micronutriments, extraits végétaux, fibres, dérivés de l'un quelconque des excipients précédents, ou des combinaisons de ceux-ci.

9. Procédé selon l'une quelconque des revendications précédentes 1 à 8, dans lequel la composition est une poudre soluble.

10. Procédé selon l'une quelconque des revendications précédentes 1 à 9, dans lequel la composition est une boisson pour sportifs.
